# EUROPÄISCHE PATENTANMELDUNG

(11) **EP 2 893 935 A1**
(43) Veröffentlichungstag der Anmeldung: **15.07.2015**
(21) Anmeldenummer: 14197338.8
(22) Anmeldetag: 11.12.2014
(51) Int. Cl.: A61K 36/74, A61K 36/23, A61K 36/28, A61K 36/48, A61P 31/00, A61P 31/04, A61K 36/185, A61K 36/70, A61K 36/704

(54) **Pharmazeutische Zusammensetzung zur Behandlung von Borreliose**

(30) Priorität: 08.01.2014 DE 102014200109
(71) Anmelder: Andres-Fischer, Wolfgang, 82110 Germering (DE)
(72) Erfinder: Andres-Fischer, Wolfgang, 82110 Germering (DE)
(74) Vertreter: Becker Kurig Straus

(57) **Zusammenfassung**

Die vorliegende Erfindung stellt eine Zusammensetzung zur Verwendung in der Behandlung der Borreliose bereit, sowie ein Kit, das die Zusammensetzung umfasst. Die erfindungsgemäße Zusammensetzung umfasst vier Gruppen an Bestandteilen, wobei Gruppe A) 1000 - 5000 mg Katzenkralle, 1000 - 3000 mg Krillöl oder Kalamarinöl oder Kombinationen davon, 200 - 600 mg Magnesium Malat, 3000 - 5000 mg Ester C, 100 - 300 mg Alpha-Liponsäure, 25 - 75 mg Pyridoxal-5-phosphat (Vitamin B6), 6000 - 14000 IU Cholecalciferol (Vitamin D3), 2000 - 4000 mg He Shou Wu, 1200 - 2500 mg Rotklee Tee, 10 - 50 ml Petersiliensaft umfasst, Gruppe B) 2000 - 4000 mg Monolaurin, 400 - 800 mg N-Acetylcystein, 100 - 300 mg Hyaluronsäure mit BioCell Kollagen II und 1 - 7 Tropfen Karde Wurzel, Gruppe C) 10 - 70 Tropfen Spilanthes Acmella umfasst und Gruppe D) 500 - 1500 µg Cyanocobalamin (Vitamin B 12) umfasst. Die Zusammensetzung und das Kit eignen sich zur Verwendung in der Behandlung von Borreliose, wobei die Bestanteile der Gruppe A) einmal täglich verabreicht werden, die der Gruppe B) zweimal täglich, das der Gruppe C) dreimal täglich und das der Gruppe D) alle 14 Tage.

## Beschreibung

Borrelien sind eine Gattung gramnegativer und äußerst schlanker Bakterien mit mehreren weiten Windungen. Benannt sind sie nach dem französischen Bakteriologen Amedee Borrel (1867-1936) und sie gehören zur Familie der Spirochäten. Ihre Windungen bzw. Spiralen sind unregelmäßig, flexibel und beweglich. Insbesondere diese Flexibilität unterscheidet Borrelien von den Spirillen.

Infektionskrankheiten, die durch Bakterien aus der Gruppe der Borrelien ausgelöst werden, werden allgemein als Borreliosen bezeichnet. Die Erkrankungen kommen beim Menschen und bei allen anderen Säugetieren sowie Vögeln vor und können durch den Befall aller Körpergewebe vielfältige klinische Symptome auslösen. Humanmedizinisch bedeutsam sind die Erregerarten *Borrelia burgdorferi sensu stricto, B. garinii*, *B. afzelii* und *B. spielmanii* als Erreger der Lyme-Borreliose (oder auch Lyme-Krankheit), sowie *B. recurrentis* und *B. duttonii*, die das Rückfallfieber auslösen können.

Eine Übertragung der Borrelioseerreger erfolgt üblicherweise durch Zecken und Läuse, die den Erreger zuvor von Rotwild und kleinen wildlebenden Nagern wie beispielsweise Mäusen oder Igeln erhalten haben. Bei den zahlreichen Zeckenarten stellen die Schildzecken *Ixodes ricinus* (Gemeiner Holzbock) in Europa und *Ixodes scapularsi* sowie *Ixodes pacificus* in Nordamerika und *Ixodes persulcatus* in Asien die Hauptvektoren für Infektionen des Menschen dar, da diese Arten euryphag sind, d.h. sie sind an einer Vielzahl von unterschiedlichen Wirten parasitierend. Je nach Region sind bis zu 30%, teilweise auch bis zu 50% der Zecken mit B. burgdoferi infiziert. Übertragungen durch Stechmücken oder Pferdebremsen wurden ebenfalls beschrieben (Leitlinie *Neuroborreliose* der Deutschen Gesellschaft für Neurologie; in AWMF online, Stand 2008).

Die Lyme-Borreliose ist in den gemäßigten Breiten die häufigste durch Arthropoden übertragene Infektionskrankheit. In Deutschland kommt es jährlich zu schätzungsweise 60.000 Neuerkrankungen.

Der klinische Verlauf der Lyme-Borreliose kann in drei Stadien unterteilt werden. Zwischen Infektion und der klinischen Manifestation können Tage bis sogar Jahre vergehen. Im ersten Stadium (Lokalinfektion), wenige Tage bis Wochen nach der Infektion, bildet sich eine von der Eintrittsstelle ausgehende und in die Umgebung vordringende konzentrische Hautrötung, die sogenannte Wanderröte (Erythema migrans). Dieses erste Stadium kann folgenlos ausheilen oder primär symptomlos verlaufen, so dass sich die Erkrankung erst im zweiten oder dritten Stadium manifestiert.

Im zweiten Stadium (Streuung des Erregers) sind vornehmlich die Haut, das zentrale und periphere Nervensystem, das Herz sowie der Bewegungsapparat betroffen. Es können aber auch die Leber, die Milz, die Lungen und die Hoden befallen werden. In Europa ist die Meningopolyneuritis Garin-Bujadoux-Bannwarth (Neuroborreliose) die häufigste klinische Manifestation. Wochen bis Monate nach dem Zeckenstich ist das Krankheitsbild vor allem von ausstrahlenden Schmerzen geprägt, die den anderen Symptomen in diesem Stadium vorausgehen, vor allem nachts. Beschrieben sind auch Ausfälle von Hirnnerven, besonders des Nervus facialis, sowie eine Beteiligung des Herzens in Form von Rhythmusstörungen, Gelenk- und Muskelschmerzen, sowie Müdigkeit und ein deutliches Krankheitsgefühl, die häufig auch mit Grippesymptomen gleichgesetzt werden.

Im dritten Stadium (Spätstadium) tritt ein chronisches Krankheitsbild meist Monate bis Jahre nach der Infektion auf und ist vor allem durch die pathognomonische Acrodermatitis chronica atrophicans (ACA) und durch rheumatologische Beschwerden gekennzeichnet. Die chronische Neuroborreliose zeichnet sich durch eine chronische Meningitis oder Enzephalomyelitis mit lymphozytärer Pleozytose im Liquor von mehr als sechs Monaten Dauer aus. Etwa die Hälfte der unbehandelten Patienten entwickelt im weiteren Krankheitsverlauf Gelenkentzündungen (sogenannte Lyme-Arthritis) typischerweise in den großen Gelenken der unteren Extremitäten. Darüber hinaus sind auch Entzündungen des Schulter- und Ellenbogengelenks sowie gerade in Europa Befälle der kleinen Fingergelenke bekannt.

Eine *B. burdorferi* Infektion hinterlässt nicht zuverlässig eine Immunität, so dass Reinfektionen vorkommen können. Im Laufe der Erkrankung werden sowohl spezifische Antikörper als auch T Zellen gebildet, wobei die Antikörper durch beispielsweise indirekte Immunfluoreszenz oder Westernblot und ELISA im Labor nachgewiesen werden können. Aufgrund der hohen Fehlerhäufigkeit bei der Bestimmung der Infektion wird in der Regel ein Zwei-Stufen-Test durchgeführt. Es wird erst ein ELISA-Test eingesetzt, der jedoch Kreuzreaktionen bzw. polyklonale Antikörperstimmulierungen durch andere Krankheitserreger aufweisen und deshalb falsch-positiv sein kann. Das Ergebnis wird dann durch einen Immuno- bzw. Westernblot verifiziert und bestätigt. Bei negativem ELISA und fortbestehendem klinischen Verdacht auf eine Borreliose empfiehlt sich die Durchführung eines Westernblot.

Die bisherige Behandlung der Borreliose ist auf die lange Gabe (mindestens vier Wochen) von Antibiotika beschränkt. *B. burgdorferi* beispielsweise zeigt sich empfindlich gegenüber β-Laktamen und Tetrazyklinen (Hahn, Falke, Kaufmann, Ullmann: "Medizinische Mikrobiologie und Infektiologie"; 4. Auflage, Springer Verlag). Da gerade in der Anfangszeit einer Infektion außer der Wanderröte kein sicherer Krankheitsnachweis möglich ist, stellt sich beim Auftreten von unspezifischen grippeähnlichen Symptomen oder Gelenkschmerzen nach einem Zeckenstich die Frage einer Güterabwägung zwischen den Risiken und Nebenwirkungen einer auf Verdacht durchgeführten, eventuell überflüssigen mehrwöchigen Antibiotikatherapie einerseits und andererseits - bei Nichtdurchführung, aber auch einem denkbaren Misserfolg einer solchen Maßnahme - den möglichen gesundheitlichen, sozialen und finanziellen Folgen eines jahrelangen chronischen Leidens, das im Extremfall bis hin zur Erwerbsunfähigkeit führen kann.

Es ist daher eine Aufgabe der vorliegenden Erfindung eine neue, von Antibiotika im Wesentlichen unabhängige Zusammensetzung zur Behandlung der Borreliose bereitzustellen.

### Zusammenfassung der Erfindung

Die vorliegende Erfindung stellt eine Zusammensetzung zur Verwendung in der Behandlung von Borreliose bereit. Die Zusammensetzung gliedert sich in drei Gruppen an Bestandteilen, wobei die Gruppe A) 1000 - 5000 mg Katzenkralle, 1000 - 3000 mg Krillöl oder Kalamarinöl oder Kombinationen davon, 200 - 600 mg Magnesium Malat, 3000 - 5000 mg Ester C, 100 - 300 mg Alpha-Liponsäure, 25 - 75 mg Pyridoxal-5-phosphat (Vitamin B6), 6000 - 14000 IU Cholecalciferol (Vitamin D3), 2000 - 4000 mg He Shou Wu, 1200 - 2500 mg Rotklee Tee und 10 - 50 ml Petersiliensaft umfasst, die Gruppe B) 2000 - 4000 mg Monolaurin, 400 - 800 mg N-Acetylcystein, 100 - 300 mg Hyaluronsäure mit BioCell Kollagen II und 1 - 7 Tropfen Karde Wurzel, die Gruppe C) 10 - 70 Tropfen Spilanthes Acmella und die Gruppe D) 500 - 1500 µg Cyanocobalamin (Vitamin B12) umfasst. Die Bestandteile der Gruppe A) werden einmal täglich verabreicht, die Bestandteile der Gruppe B) werden zweimal täglich verabreicht, der Bestandteil der Gruppe C) wird dreimal täglich verabreicht und der Bestandteil der Gruppe D) einmal in 14 Tagen.

In einer bevorzugten Ausführungsform ist die Borreliose eine Lyme-Borreliose.

Monolaurin, N-Acetylcystein, Hyaluronsäure mit BioCell Kollagen II und Karde Wurzel (Gruppe B)) werden vorzugsweise morgens und abends verabreicht. Besonders bevorzugt liegen zwischen den zwei Verabreichungen zwischen 15 und 6 Stunden, mehr bevorzugt zwischen 13 und 8 Stunden und am meisten bevorzugt zwischen 12 und 10 Stunden.

Spilanthes Acmella (Gruppe C)) wird vorzugsweise morgens, mittags und abends verabreicht. Besonders bevorzugt liegen zwischen den drei Verabreichungen zwischen 14 und 2 Stunden, mehr bevorzugt zwischen 11 und 4 Stunden und am meisten bevorzugt zwischen 8 und 6 Stunden.

Cyanocobalamin (Gruppe D)) wird vorzugsweise am ersten, zweiten, dritten, vierten, fünften, sechsten, siebten, achten, neunten, zehnten, elften, zwölften, dreizehnten oder vierzehnten Tag nach der ersten Verabreichung der Bestandteile der Gruppen A), B) und C) verabreicht.

Die Zusammensetzung wird vorzugsweise oral verabreicht. Alternativ kann die Zusammensetzung auch intravenös verabreicht werden. Es können alle Stoffe gleichzeitig oder sukzessive verabreicht werden. Cyanocobalamin wird vorzugsweise durch Injektion verabreicht, bevorzugt durch subkutane, intramuskuläre oder intravenöse Injektion.

In einer bevorzugten Ausführungsform umfasst die Zusammensetzung in Gruppe A) 1500 - 4500 mg Katzenkralle, 1500 - 2500 mg Krillöl oder Kalamarinöl oder Kombinationen davon, 300 - 500 mg Magnesium Malat, 3500 - 4500 mg Ester C, 150 - 250 mg Alpha-Liponsäure, 40 - 60 mg Pyridoxal-5-phosphat (Vitamin B6), 8000 - 12000 IU Cholecalciferol (Vitamin D3), 2500 - 3500 mg He Shou Wu, 1500 - 2250 mg Rotklee Tee, 20 - 40 ml Petersiliensaft, in Gruppe B) 2500 - 3500 mg Monolaurin, 500 - 700 mg N-Acetylcystein, 150 - 250 mg Hyaluronsäure mit BioCell Kollagen II und 1 - 4 Tropfen Karde Wurzel, in Gruppe C) 20 - 60 Tropfen Spilanthes Acmella und in Gruppe D) 800 - 1200 µg Cyanocobalamin (Vitamin B12).

Besonders bevorzugt umfasst die Zusammensetzung in Gruppe A) 2000-4000 mg Katzenkralle, 2000 mg Krillöl oder Kalamarinöl oder Kombinationen davon, 400 mg Magnesium Malat, 4000 mg Ester C, 200 mg Alpha Liponsäure, 50 mg Pyridoxal-5-phosphat, 10.000 IU Cholecalciferol, 3000 mg He Shou Wu, 1500-2250 mg Rotklee Tee, 30 ml Petersiliensaft, in Gruppe B) 3000 mg Monolaurin, 600 mg N-Acetylcystein, 200 mg Hyaloronsäure mit BioCell Kollagen II und 2 - 3 Tropfen Karde Wurzel, in Gruppe C) 40 Tropfen Spilanthes Acmella und in Gruppe D) 1000 µg Cyanocobalamin (Vitamin B12).

Die vorliegende Erfindung stellt ebenfalls ein Kit bereit, das die folgenden Gruppen an Bestandteilen umfasst: Gruppe A) mit 1000 - 5000 mg Katzenkralle, 1000 - 3000 mg Krillöl oder Kalamarinöl oder Kombinationen davon, 200 - 600 mg Magnesium Malat, 3000 - 5000 mg Ester C, 100 - 300 mg Alpha-Liponsäure, 25 - 75 mg Pyridoxal-5-phosphat (Vitamin B6), 6000 - 14000 IU Cholecalciferol (Vitamin D3), 2000 - 4000 mg He Shou Wu, 1200 - 2500 mg Rotklee Tee, 10 - 50 ml Petersiliensaft, Gruppe B) mit 2000 - 4000 mg Monolaurin, 400 - 800 mg N-Acetylcystein, 100 - 300 mg Hyaluronsäure mit BioCell Kollagen II und 1 - 7 Tropfen Karde Wurzel, Gruppe C) mit 10 - 70 Tropfen Spilanthes Acmella und Gruppe D) mit 500 - 1500 µg Cyanocobalamin (Vitamin B12), sowie eine Anleitung zur Anwendung bzw. Verabreichung der einzelnen Gruppen A)-D).

In einer bevorzugten Ausführungsform umfasst das Kit die folgenden Gruppen an Bestandteilen: Gruppe A) 1500 - 4500 mg Katzenkralle, 1500 - 2500 mg Krillöl oder Kalamarinöl oder Kombinationen davon, 300 - 500 mg Magnesium Malat, 3500 - 4500 mg Ester C, 150 - 250 mg Alpha-Liponsäure, 40 - 60 mg Pyridoxal-5-phosphat (Vitamin B6), 8000 - 12000 IU Cholecalciferol (Vitamin D3), 2500 - 3500 mg He Shou Wu, 1500 - 2250 mg Rotklee Tee, 20 - 40 ml Petersiliensaft, Gruppe B) 2500 - 3500 mg Monolaurin, 500 - 700 mg N-Acetylcystein, 150 - 250 mg Hyaluronsäure mit BioCell Kollagen II und I - 4 Tropfen Karde Wurzel, Gruppe C) 20 - 60 Tropfen Spilanthes Acmella und Gruppe D) 800 - 1200 µg Cyanocobalamin (Vitamin B12).

In einer besonders bevorzugten Ausführungsform umfasst das Kit die folgenden Gruppen an Bestandteilen: Gruppe A) 2000 - 4000 mg Katzenkralle, 2000 mg Krillöl oder Kalamarinöl oder Kombinationen davon, 400 mg Magnesium Malat, 4000 mg Ester C, 200 mg Alpha Liponsäure, 50 mg Pyridoxal-5-phosphat, 10.000 IU Cholecalciferol, 3000 mg He Shou Wu, 1500 - 2250 mg Rotklee Tee, 30 ml Petersiliensaft, Gruppe B) 3000 mg Monolaurin, 600 mg N-Acetylcystein, 200 mg Hyaloronsäure mit BioCell Kollagen II und 2 - 3 Tropfen Karde Wurzel, Gruppe C) 40 Tropfen Spilanthes Acmella und Gruppe D) 1000 µg Cyanocobalamin (Vitamin B 12).

Die Anleitung des erfindungsgemäßen Kits kann eine Anleitung zur Behandlung von Borreliose umfassen und aufführen, dass die Bestanteile der Gruppe A) einmal täglich verabreicht werden sollen, die der Gruppe B) zweimal täglich, die der Gruppe C) dreimal täglich und das der Gruppe D) alle 14 Tage.

Das erfindungsgemäße Kit eignet sich zur Behandlung der Borreliose, bevorzugt der Lyme-Borreliose.

In dem erfindungsgemäßen Kit kann die Anleitung aufführen, dass die Bestandteile der Gruppe B) morgens und abends verabreicht werden sollen.

In dem erfindungsgemäßen Kit kann die Anleitung aufführen, dass der Bestandteil der Gruppe C) morgens, mittags und abends verabreicht werden sollen.

In dem erfindungsgemäßen Kit kann die Anleitung aufführen, dass das Bestandteil der Gruppe D) am ersten, zweiten, dritten, vierten, fünften, sechsten, siebten, achten, neunten, zehnten, elften, zwölften, dreizehnten oder vierzehnten Tag nach der primären Verabreichung der Bestandteile der Gruppen A), B) und C) verabreicht werden soll.

### Detaillierte Beschreibung der Erfindung

Die vorliegende Erfindung stellt eine Zusammensetzung bereit, die sich bei der Behandlung von Borreliose, vorzugsweise Lyme-Borreliose als äußerst wirksam erwiesen hat. Die einzelnen Bestandteile der Zusammenfassung lassen sich in vier Gruppen A) bis D) gliedern, wobei die die Gruppe A) Katzenkralle, Krillöl oder Kalamarinöl oder Kombinationen davon, Magnesium Malat, Ester C, Alpha-Liponsäure, Pyridoxal-5-phosphat (Vitamin B6), Cholecalciferol (Vitamin D3), He Shou Wu, Rotklee Tee und Petersiliensaft aufweist, die Gruppe B) Monolaurin, N-Acetylcystein, Hyaluronsäure mit BioCell Kollagen II und Karde Wurzel, die Gruppe C) Spilanthes Acmella und die Gruppe D) Cyanocobalamin (Vitamin B12).

Die Bestandteile der Gruppe A) werden einmal täglich verabreicht, die Bestandteile der Gruppe B) werden zweimal täglich verabreicht, der Bestandteil der Gruppe C) wird dreimal täglich verabreicht und der Bestandteil der Gruppe D) einmal binnen 14 Tagen.

Die Menge an Katzenkralle in der Zusammensetzung beträgt 1000 - 5000 mg, bevorzugt 1500 - 4500 mg, mehr bevorzugt 1750 - 4250 mg, noch mehr bevorzugt 1900 - 4100 mg und am meisten bevorzugt 2000 - 4000 mg. Katzenkralle (engl.: Cat's Claw, wissenschaftlicher Name *Uncaria tomentosa*) kann von Natural Organics, Inc., 548 Broadhollow Road, Melville, NY 11747, USA, bezogen werden. Katzenkralle ist ein Pflanzenextrakt in Kapselform, welcher aus der inneren Baumrinde gewonnen wird. Die hier aufgeführten Mengenangaben beziehen sich auf Katzenkralle von Natural Organics, Inc..

Die Menge an Krillöl oder Kalamarinöl oder Kombinationen davon in der Zusammensetzung beträgt 1000 - 3000 mg, bevorzugt 1250 - 2750 mg, mehr bevorzugt 1500 - 2500 mg, noch mehr bevorzugt 1750 - 2250 mg und am meisten bevorzugt 2000 mg. Krillöl bzw. Kalamarinöl kann von Greenvitamins N.V., Kruisdonk 66, NL-6222 PH Maastricht, Niederlande, bezogen werden. Die hier aufgeführten Mengenangaben beziehen sich auf das Krillöl bzw. Kalamarinöl von Greenvitamins.

Die Menge an Magnesium Malat in der Zusammensetzung beträgt 200 - 600 mg, bevorzugt 300 - 500 mg, mehr bevorzugt 350 - 450 mg, noch mehr bevorzugt 375 - 425 mg und am meisten bevorzugt 400 mg.

Die Menge an Ester C in der Zusammensetzung beträgt 3000 - 5000 mg, bevorzugt 3500 - 4500 mg, mehr bevorzugt 3750 - 4250 mg, noch mehr bevorzugt 3900 - 4100 mg und am meisten bevorzugt 4000 mg. Ester C ist in Form von Tabletten von Solgar Inc., 500 Willow Tree Road, Leonia, NJ 07605, USA, erhältlich. Eine 500 mg Tablette enthält 500 mg Vitamin C als Ester C-Calcium Ascorbat, 60 mg Calcium als Calcium Ascorbat, 25 mg Citrus Bioflavonoid Komplex, 10 mg Acerola, 10 mg Frucht der Hagebutte und 5 mg Rutin. Diese Inhaltsangaben beziehen sich auf Angaben von Solar Inc.. Die hier aufgeführten Mengenangaben in Bezug auf die vorliegende Erfindung beziehen sich auf das Ester C von Solgar Inc..

Die Menge an Alpha-Liponsäure in der Zusammensetzung beträgt 100 - 300 mg, bevorzugt 150 - 250 mg, mehr bevorzugt 170 - 220 mg, noch mehr bevorzugt 190 - 210 mg und am meisten bevorzugt 200 mg.

Die Menge an Pyridoxal-5-phosphat (Vitamin B6) in der Zusammensetzung beträgt 25 - 75 mg, bevorzugt 30 - 70 mg, mehr bevorzugt 40 - 60 mg, noch mehr bevorzugt 45 - 55 mg und am meisten bevorzugt 50 mg.

Die Menge an Cholecalciferol (Vitamin D3) in der Zusammensetzung beträgt 6000 - 14000 IU, bevorzugt 8000 - 12000 IU, mehr bevorzugt 9000 - 11000 IU, noch mehr bevorzugt 9500 - 10500 IU und am meisten bevorzugt 10000 IU.

Die Menge an He Shou Wu in der Zusammensetzung beträgt 2000 - 4000 mg, bevorzugt 2500 - 3500 mg, mehr bevorzugt 2750 - 3250 mg, noch mehr bevorzugt 2900 - 3100 mg und am meisten bevorzugt 3000 mg. He Shou Wu, auch als Fo-Ti in der traditionellen chinesischen Medizin bekannt, wird aus der Wurzel von *Polygonum multi florum* gewonnen und kann von beispielsweise von PureRaw, Lockstedter Chaussee 1, 38486 Klötze, Deutschland, in Form eines Pulvers bezogen werden, auf das sich die hier aufgeführten Mengenangaben beziehen. He Shou Wu kann auch bezogen werden von : BodyMe Ltd, Unit 12 Rockhill Estate, Wellsway, Keynsham, Bristol, BS31 1PF, Großbritannien.

Die Menge an Rotklee Tee in der Zusammensetzung beträgt 1200 - 2500 mg, bevorzugt 1500 - 2250 mg, mehr bevorzugt 1750 - 2000 mg, noch mehr bevorzugt 1800 - 1900 mg und am meisten bevorzugt 1850 mg. Rotklee Tee besteht aus Rotkleeblüten und Grobschnitt und kann beispielsweise von AURICA Naturheilmittel und Naturwaren GmbH, Püttlinger Str. 121, 66773 Schwalbach, Deutschland, bezogen werden. Die hier aufgeführten Mengenangaben beziehen sich auf den Rotklee Tee von AURICA.

Die Menge an Petersiliensaft in der Zusammensetzung beträgt 10 - 50 ml, bevorzugt 20 - 40 ml, mehr bevorzugt 25 - 35 ml, noch mehr bevorzugt 27 - 32 ml und am meisten bevorzugt 30 ml. Petersiliensaft kann in Form eines Petersilienkraut-Presssafts von der Walther Schoenenberger Pflanzensaftwerk GmbH & Co. KG, Hutwiesenstraße 14, 71106 Magstadt, Deutschland, bezogen werden. Die hier aufgeführten Mengenangaben beziehen sich auf den Petersiliensaft der Walther Schoenenberger Pflanzensaftwerk GmbH & Co. KG.

Die Menge an Monolaurin in der Zusammensetzung beträgt 2000 - 4000 mg, bevorzugt 2500 - 3500 mg, mehr bevorzugt 2750 - 3250 mg, noch mehr bevorzugt 2900 - 3100 mg und am meisten bevorzugt 3000 mg. Monolaurin kann von Aviva Natural Health Solutions, 1224 St. JamesStreet, Winnipeg, MB Canada R3H 0L1, bezogen werden. Die hier aufgeführten Mengenangaben beziehen sich auf Monolaurin von Aviva Natural Health Solutions.

Die Menge an N-Acetylcystein in der Zusammensetzung beträgt 400 - 800 mg, bevorzugt 500 - 700 mg, mehr bevorzugt 550 - 650 mg, noch mehr bevorzugt 575 - 625 mg und am meisten bevorzugt 600 mg.

Die Menge an Hyaluronsäure mit BioCell Kollagen II in der Zusammensetzung beträgt 100 - 300 mg, bevorzugt 150 - 250 mg, mehr bevorzugt 175 - 225 mg, noch mehr bevorzugt 180 - 220 mg und am meisten bevorzugt 200 mg. Hyaluronsäure mit BioCell Kollagen II kann von Vitabay B.V., Avenue Ceramique 221, Maastricht, NL-6221 KX, Niederlande, bezogen werden. Die hier aufgeführten Mengenangaben beziehen sich auf Hyaluronsäure mit BioCell Kollagen II von Vitabay B.V..

Die Menge an Karde Wurzel in der Zusammensetzung beträgt 1 - 7 Tropfen, bevorzugt 1 - 6 Tropfen, mehr bevorzugt 1 - 5 Tropfen, noch mehr bevorzugt 1 - 4 Tropfen und am meisten bevorzugt 2 - 3 Tropfen. Karde Wurzel kann beispielsweise von Crescent Moon Herbals LLC, c/o Mary Lavoie, Proprietress, 76 Center Road, Lebanon, ME 04027, USA bezogen werden. Karde Wurzel ist eine Tinktur und wird in einem Verhältnis von 2:1 mit destilliertem 40% Kornalkohol hergestellt. Die hier aufgeführten Mengenangaben beziehen sich auf dieses Produkt. Karde Wurzel kann auch bezogen werden von : Nuhrovia GmbH, Birkenweg 9, A-5145 Neukirchen, Österreich.

Die Menge an Spilanthes Acmella in der Zusammensetzung beträgt 10 - 70 Tropfen, bevorzugt 20 - 60 Tropfen, mehr bevorzugt 30 - 50 Tropfen, noch mehr bevorzugt 35 - 45 Tropfen und am meisten bevorzugt 40 Tropfen. Spilanthes Acmella kann als Pflanzenextrakt aus dem frisch blühenden Pflanzenkraut bzw. der Pflanzenwurzel von Herb Pharm, 20260 Williams Hwy., Williams, Oregon 97544, USA bezogen werden. Die hier aufgeführten Mengenangaben beziehen sich auf dieses Produkt.

Die Menge an Cyanocobalamin (Vitamin B 12) in der Zusammensetzung beträgt 500 - 1500 µg, bevorzugt 600 - 1400 µg, mehr bevorzugt 800 - 1200 µg, noch mehr bevorzugt 900 - 1100 µg und am meisten bevorzugt 1000 µg.

Magnesium Malat, Alpha-Liponsäure, Vitamine B6, B12, D3 sowie N-Acetylcystein sind in Deutschland frei im Handel oder auch in Apotheken erhältlich.

Die vorliegende Erfindung eignet sich insbesondere als Ersatz der prophylaktischen Antibiotikatherapie bei Borrelioseverdacht, da bisher keine Nebenwirkungen in Verbindung mit den einzelnen Bestandteilen oder ihrer Kombination bekannt geworden sind.

Das erfindungsgemäße Kit umfasst alle Bestandteile der Gruppen A) bis D) sowie eine Anleitung zur Verabreichung der Gruppen A) bis D) wie vorstehend aufgeführt. Daher eignet sich das Kit insbesondere bei der Behandlung von Borreliose, vorzugsweise von Lyme-Borreliose. Die Mengen- und Ursprungsangaben der Bestandteile der einzelnen Gruppen A) bis D) der vorstehend beschriebenen Zusammensetzung gelten auch für das erfindungsgemäße Kit. Ebenso sind die dargestellten Verabreichungsformen ebenfalls auf das Kit anwendbar.

### Beispiele

Die erfindungsgemäße Zusammensetzung wurde 5 Personen verabreicht (1 männlich, 4 weiblich) im Alter von 37 bis 73 Jahren. Das durchschnittliche Alter der Patienten betrug 58 Jahre.

Die Therapie entsprach der angegebenen Zusammensetzung und bestand im Folgenden aus: oral 1 x täglich 2000 - 4000 mg Katzenkralle, 2000 mg Krillöl, 400 mg Magnesium Malat, 4000 mg Ester C, 200 mg Alpha Liponsäure, 50 mg Pyridoxal-5-phosphat, 10.000 IU Cholecalciferol, 3000 mg He Shou Wu, 1500 - 2250 mg Rotklee Tee, 30 ml Petersiliensaft; 2 x täglich 3000 mg Monolaurin, 600 mg N-Acetylcystein, 200 mg Hyaloronsäure mit BioCell Kollagen II und 2-3Tropfen Karde Wurzel; 3 x täglich 40 Tropfen Spilanthes Acmella und alle 14 Tage intramuskulär bzw. subkutan 1000 µg Cyanocobalamin. Voraussetzung war eine strikte Einhaltung der vorgegebenen Darreichungsform.

Die Ausgangssymptome der Patienten wiesen ein breites Spektrum auf, da sich die Krankheitserreger in der Spinalflüssigkeit, Gelenkflüssigkeit, Augenflüssigkeit absetzen und verstecken sowie im Blutkreislauf diffus über den Körper verteilen können.

Die Diagnosefindung erfolgte neben der Anamnese und den klinischen Symptomen anhand von Bluttests und/oder Biopsien mit Serologie (über spezifische Antikörper mit Immunoassays ELISA oder CLIA, spezifischer Immunoblot mit entsprechendem Immunoglobin- und Bandenmuster), Liquor- und/oder Gelenkpunktion mit PCR, Anzucht des Erregers *Borrelia burgdorferi* oder Bestimmung des Chemokins CXCL13). In allen Patineten konnte der Erreger *Borrelia burgdorferi* nachgewiesen werden.

Die Anwendungsdauer der Therapie betrug im Schnitt 5 Monate. Am Ende der Therapie beschrieben alle Patienten eine deutliche Befundbesserung insbesondere im Hinblick auf die Symptome. Die Erreger waren in den darauf folgenden Bluttests nicht mehr nachweisbar.

Patient 1: 37 Jahre alt, männlich. Therapiedauer 3 ½ Monate. Der Patient wies zu Beginn der Therapie die folgenden Symptome auf: Müdigkeit und Übelkeit, alterierend Kopfschmerzen, Halsschmerzen, kurze Erkältung, kein Fieber. Nach Abschluss der Therapie war der Patient beschwerdefrei.

Patient 2: 44 Jahre alt, weiblich. Therapiedauer 4 Monate. Die Patientin wies zu Beginn der Therapie die folgenden Symptome auf: Innere Unruhe, Nervosität, Schlafstörungen, Kopfschmerzen, Bauchschmerzen. Nach Abschluss der Therapie war die Patientin beschwerdefrei.

Patient 3: 71 Jahre alt, weiblich. Therapiedauer 5 Monate. Die Patientin wies zu Beginn der Therapie die folgenden Symptome auf: Wechselnde Gelenkschmerzen (Ellenbogengelenk, Handgelenk, Fußgelenk, Kniegelenk), Durchfall, Übelkeit, Verdauungsprobleme, Bauchschmerzen, Kopfschmerzen, Müdigkeit, Schlaflosigkeit, innere Unruhe, Fieber. Nach Abschluss der Therapie war die Patientin beschwerdefrei.

Patient 4: 65 Jahre alt, weiblich. Therapiedauer 5 ½ Monate. Die Patientin wies zu Beginn der Therapie die folgenden Symptome auf: Wechselnde Gelenkschmerzen, Hauterscheinungen mit Erythem, gelegentlich Kopfschmerzen, innere Unruhe, Übelkeit, gering erhöhte Fieberwerte. Nach Abschluss der Therapie war die Patientin beschwerdefrei.

Patient 5: 73 Jahre alt, weiblich. Therapiedauer 5 Monate. Die Patientin wies zu Beginn der Therapie die folgenden Symptome auf: Wechselnde Gelenkschmerzen, Gelenksdeformitäten, Gehbehinderung, Herzklappen-Insuffizienz, Neuropathie, körperliche Schwäche, wiederkehrendes Fieber, Konzentrationsdelizite. Nach Abschluss der Therapie war die Patientin beschwerdefrei.

## Patentansprüche

1. Zusammensetzung umfassend die folgenden Gruppen an Bestandteilen:
Gruppe A) mit
1000 - 5000 mg Katzenkralle
1000 - 3000 mg Krillöl oder Kalamarinöl oder Kombinationen davon 200 - 600 mg Magnesium Malat
3000 - 5000 mg Ester C
100 - 300 mg Alpha-Liponsäure
25 - 75 mg Pyridoxal-5-phosphat (Vitamin B6)
6000 - 14000 IU Cholecalciferol (Vitamin D3)
2000 - 4000 mg He Shou Wu
1200 - 2500 mg Rotklee Tee
10 - 50 ml Petersiliensaft
Gruppe B) mit
2000 - 4000 mg Monolaurin
400 - 800 mg N-Acetylcystein
100 - 300 mg Hyaluronsäure mit BioCell Kollagen II
1 - 7 Tropfen Karde Wurzel
Gruppe C) mit
10 - 70 Tropfen Spilanthes Acmella und
Gruppe D) mit
500 - 1500 µg Cyanocobalamin (Vitamin B 12)
zur Verwendung in der Behandlung von Borreliose, wobei die Bestanteile der Gruppe A) einmal täglich verabreicht werden, die der Gruppe B) zweimal täglich, das der Gruppe C) dreimal täglich und das der Gruppe D) alle 14 Tage.

2. Zusammensetzung zur Verwendung nach Anspruch 1, wobei die Borreliose Lyme-Borreliose ist.

3. Zusammensetzung zur Verwendung nach Anspruch 1 oder 2, wobei die Bestandteile der Gruppe B) morgens und abends verabreicht werden.

4. Zusammensetzung zur Verwendung nach Anspruch 1, 2 oder 3, wobei der Bestandteil der Gruppe C) morgens, mittags und abends verabreicht wird.

5. Zusammensetzung zur Verwendung nach einem der vorstehenden Ansprüche, wobei das Bestandteil der Gruppe D) am ersten, zweiten, dritten, vierten, fünften, sechsten, siebten, achten, neunten, zehnten, elften, zwölften, dreizehnten oder vierzehnten Tag nach der primären Verabreichung der Bestandteile der Gruppen A), B) und C) verabreicht wird.

6. Zusammensetzung zur Verwendung nach einem der vorstehenden Ansprüche, wobei die Bestandteile der Gruppen A), B) und C) oral verabreicht werden.

7. Zusammensetzung zur Verwendung nach einem der Ansprüche 1-6, wobei das Bestandteil der Gruppe D) durch subkutane, intramuskuläre oder intravenöse Injektion verabreicht wird.

8. Zusammensetzung zur Verwendung nach einem der vorstehenden Ansprüche, umfassend die folgenden Gruppen an Bestandteilen:
Gruppe A) mit
1500 - 4500 mg Katzenkralle
1500 - 2500 mg Krillöl oder Kalamarinöl oder Kombinationen davon
300 - 500 mg Magnesium Malat
3500 - 4500 mg Ester C
150 - 250 mg Alpha-Liponsäure
40 - 60 mg Pyridoxal-5-phosphat (Vitamin B6)
8000 - 12000 IU Cholecalciferol (Vitamin D3)
2500 - 3500 mg He Shou Wu
1500 - 2250 mg Rotklee Tee
20 - 40 ml Petersiliensaft
Gruppe B) mit
2500 - 3500 mg Monolaurin
500 - 700 mg N-Acetylcystein
150 - 250 mg Hyaluronsäure mit BioCell Kollagen II
1 - 4 Tropfen Karde Wurzel
Gruppe C) mit
20 - 60 Tropfen Spilanthes Acmella, und
Gruppe D) mit
800 - 1200 µg Cyanocobalamin (Vitamin B12).

9. Zusammensetzung zur Verwendung nach Anspruch 8, umfassend
Gruppe A) mit
2000 - 4000 mg Katzenkralle
2000 mg Krillöl oder Kalamarinöl oder Kombinationen davon
400 mg Magnesium Malat
4000 mg Ester C
200 mg Alpha-Liponsäure
50 mg Pyridoxal-5-phosphat (Vitamin B6)
10000 IU Cholecalciferol (Vitamin D3)
3000 mg He Shou Wu
1500 - 2250 mg Rotklee Tee
30 ml Petersiliensaft
Gruppe B) mit
3000 mg Monolaurin
600 mg N-Acetylcystein
200 mg Hyaluronsäure mit BioCell Kollagen II
2- 3 Tropfen Karde Wurzel
Gruppe C) mit
40 Tropfen Spilanthes Acmella und
Gruppe D) mit
1000 µg Cyanocobalamin (Vitamin B12).

10. Kit umfassend die folgenden Gruppen an Bestandteilen:
Gruppe A) mit
1000 - 5000 mg Katzenkralle
1000 - 3000 mg Krillöl oder Kalamarinöl oder Kombinationen davon
200 - 600 mg Magnesium Malat
3000 - 5000 mg Ester C
100 - 300 mg Alpha-Liponsäure
25 - 75 mg Pyridoxal-5-phosphat (Vitamin B6)
6000 - 14000 IU Cholecalciferol (Vitamin D3)
2000 - 4000 mg He Shou Wu
1200 - 2500 mg Rotklee Tee
10 - 50 ml Petersiliensaft
Gruppe B) mit
2000 - 4000 mg Monolaurin
400 - 800 mg N-Acetylcystein
100 - 300 mg Hyaluronsäure mit BioCell Kollagen II
1 - 7 Tropfen Karde Wurzel
Gruppe C) mit
10-70 Tropfen Spilanthes Acmella, und
Gruppe D) mit
500 - 1500 µg Cyanocobalamin (Vitamin B12).
sowie eine Anleitung zur Anwendung der einzelnen Gruppen A)-D).

11. Kit nach Anspruch 10, umfassend die folgenden Gruppen an Bestandteilen:
Gruppe A) mit
1500 - 4500 mg Katzenkralle
1500 - 2500 mg Krillöl oder Kalamarinöl oder Kombinationen davon
300 - 500 mg Magnesium Malat
3500 - 4500 mg Ester C
150 - 250 mg Alpha-Liponsäure
40 - 60 mg Pyridoxal-5-phosphat (Vitamin B6)
8000 - 12000 IU Cholecalciferol (Vitamin D3)
2500 - 3500 mg He Shou Wu
1500 - 2250 mg Rotklee Tee
20 - 40 ml Petersiliensaft
Gruppe B) mit
2500 - 3500 mg Monolaurin
500 - 700 mg N-Acetylcystein
150 - 250 mg Hyaluronsäure mit BioCell Kollagen II und
1 - 4 Tropfen Karde Wurzel
Gruppe C) mit
20 - 60 Tropfen Spilanthes Acmella, und
Gruppe D) mit
800 - 1200 µg Cyanocobalamin (Vitamin B 12).

12. Kit nach Anspruch 11, umfassend die folgenden Gruppen an Bestandteilen:
Gruppe A) mit
2000 - 4000 mg Katzenkralle
2000 mg Krillöl oder Kalamarinöl oder Kombinationen davon
400 mg Magnesium Malat
4000 mg Ester C
200 mg Alpha-Liponsäure
50 mg Pyridoxal-5-phosphat (Vitamin B6)
10000 IU Cholecalciferol (Vitamin D3)
3000 mg He Shou Wu
1500 - 2250 mg Rotklee Tee
30 ml Petersiliensaft
Gruppe B) mit
3000 mg Monolaurin
600 mg N-Acetylcystein
200 mg Hyaluronsäure mit BioCell Kollagen II
2 - 3 Tropfen Karde Wurzel
Gruppe C) mit
40 Tropfen Spilanthes Acmella und
Gruppe D) mit
1000 µg Cyanocobalamin (Vitamin B 12).

13. Kit nach einem der Ansprüche 10-12, worin die Anleitung Anweisungen zur Behandlung von Borreliose umfasst und aufführt, dass die Bestanteile der Gruppe A) einmal täglich verabreicht werden sollen, die der Gruppe B) zweimal täglich, das der Gruppe C) dreimal täglich und das der Gruppe D) alle 14 Tage.

14. Kit nach Anspruch 13, worin die Borreliose Lyme-Borreliose ist.

15. Kit nach Anspruch 13 oder 14, worin die Anleitung aufführt, dass die Bestandteile der Gruppe B) morgens und abends verabreicht werden sollen, und/oder der Bestandteil der Gruppe C) morgens, mittags und abends verabreicht werden soll, und/oder der Bestandteil der Gruppe D) am ersten, zweiten, dritten, vierten, fünften, sechsten, siebten, achten, neunten, zehnten, elften, zwölften, dreizehnten oder vierzehnten Tag nach der primären Verabreichung der Bestandteile der Gruppen A), B) und C) verabreicht werden soll.
